(19) Europäisches Patentamt
European Patent Office
Office européen des brevets

(11) **EP 3 618 079 A1**

(12) **EUROPEAN PATENT APPLICATION**

(43) Date of publication:
**04.03.2020 Bulletin 2020/10**

(51) Int Cl.:
**G16H 40/63** *(2018.01)*          **G16H 30/20** *(2018.01)*
**G16H 20/10** *(2018.01)*          **G16H 20/40** *(2018.01)*

(21) Application number: **18191668.5**

(22) Date of filing: **30.08.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **LEUSSLER, Christoph**
  **5656 AE Eindhoven (NL)**

• **HELLE, Michael**
  **5656 AE Eindhoven (NL)**
• **WIRTZ, Daniel**
  **5656 AE Eindhoven (NL)**
• **VOGTMEIER, Gereon**
  **5656 AE Eindhoven (NL)**
• **JOHNSON, Mark Thomas**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **IMAGING METHOD USING PAIN SENSOR**

(57)    A system for controlling operation of an imaging or therapy apparatus. The system comprises an interface (IN) for receiving a pain measurement signal as measured by one or more sensors (S) in relation to an anatomic part (BR) of the patient (PAT) i) being imaged in an imaging procedure by the imaging apparatus or ii) being under therapy in a therapy procedure delivered by the therapy device, whilst the part (BR) is held in an adjustable fixation device (FD). A control unit (CU) of the system is configured to process the pain measurement signal to compute at least one control signal. A control interface (CIF) of the system is configured to interact during the imaging or therapy procedure with the imaging apparatus (IA) and/or the fixation device (FD) based on the control signal to a) influence the imaging or therapy procedure and/or b) to adjust the fixation device so as to change the manner in which the part (BR) is being held.

Fig.1

**Description**

FIELD OF THE INVENTION

The invention relates to a system of supporting imaging, an imaging arrangement, to a method of supporting imaging, to a method of training a machine learning component, to a computer program element, to a computer readable medium, and to a fixation device.

BACKGROUND OF THE INVENTION

Breast cancer remains a leading cause of death in women.

Overall, mammography is considered the single most effective screening tool and has been credited with reducing breast cancer-related mortality.

There is no doubt that mammography is a very cost-effective tool for breast cancer screening. Breast MRI is currently *the* most sensitive detection technique for breast cancer diagnosis. It is able to detect cancer not visible on conventional imaging (such as X-ray based), it can be used as a problem-solving instrument, and it can be applied to screen high-risk patients. Breast MRI is also better at monitoring the response to chemotherapy than other imaging modalities used today.

There exist a range of MRI solutions for diagnostic imaging of the mamma.

Diagnostic imaging of the mamma requires same to remain stationary to prevent image degradation which may otherwise result from motion of the region of interest.

Fixation of the mamma, or indeed of other body parts, can be a painful experience for the patient resulting.

SUMMARY OF THE INVENTION

There may therefore be a need to improve imaging, in particular of regions of interest that require fixation.

The object of the present invention is solved by the subject matter of the independent claims where further embodiments are incorporated in the dependent claims. It should be noted that the following described aspect of the invention equally applies to the system of supporting imaging, to the imaging arrangement, to the method of supporting imaging, to the computer program element, to the computer readable medium, and to the fixation device.

According to one aspect there is provided a system for supporting operation of an imaging or therapy apparatus, comprising:

- an interface for receiving a pain measurement signal as measured by one or more sensors in relation to an anatomic part of the patient i) being imaged in an imaging procedure by the imaging apparatus or ii) being under therapy in a therapy procedure delivered by the therapy device, whilst the part is held in an adjustable fixation device;
- a control unit configured to process the pain measurement signal to compute at least one control signal; and
- a control interface configured to interact during the imaging or therapy procedure with i) the imaging apparatus or therapy device and/or ii) the fixation device based on the control signal to a) influence the imaging procedure or therapy procedure and/or b) to adjust the fixation device so as to change the manner in which the part is being held.

In embodiments, the control unit is configured to take into account at least one pre-set image quality or therapy parameter when computing the control signal.

In embodiments, the control unit is configured to compute the control signal so as to at least maintain the imaging or therapy quality as per the pre-set image or therapy quality parameter.

In embodiments, the control unit is configured in a feedback architecture in relation to a pain threshold for the received pain measurement signal and/or the preset image or therapy quality parameter. The pain threshold and the pre-set image or therapy quality parameter maybe set as respective setpoints of the feedback architecture. In embodiments, two feedback loops are used, one for the pain level monitoring and one for the image or therapy quality monitoring. Preferably the two loops are linked. Preferably the link is through the fixation device control signal which is provided as output through the pain level measurement loop and as input for the image quality of therapy quality loop.

In embodiments, the imaging procedure includes acquisition of image data.

In embodiments, the influencing of the imaging procedure includes i) suspending or resuming the acquisition of image data or ii) suspending or resuming therapy delivery.

In embodiments, the fixation device is adjustable so as to change a pressure distribution exerted by the fixation device on at least a portion of the part.

In embodiments, the fixation device includes an adjustable contact surface urgeable into contact with at least a portion of the part, wherein the said contact surface shape is adjustable so as to conform to at least a shape of a portion of the part.

In embodiments, the contact surface shape is adjustable in 3D. In embodiments, this is achieved by having the contact surface formed by an array of individually addressable and actuatable contact elements.

In embodiments, the control unit is configurable to operate in a pain locator mode so as to determine, based on the pain measurement signal, where in or on the part a pain is caused.

In embodiments, the imaging apparatus is any one of: an MRI imager, an X-ray imager; an emission imager, a linac system.

In embodiments, the system comprises a pre-trained machine learning component included in or, coupled to, the control unit.

In a further aspect there is provided an imaging arrangement, comprising: the system of one of the previous claims, the imaging apparatus, the at least one sensor.

In a further aspect there is provided a method of supporting imaging or therapy, comprising:

- receiving a pain measurement signal as measured by one or more sensors in relation to a part of the patient i) being imaged in an imaging procedure by the imaging apparatus or ii) being under therapy in a therapy procedure delivered by the therapy device, whilst the is held in an adjustable fixation device;
- processing the pain measurement signal to compute at least one control signal; and
- interacting during the procedure with the i) imaging apparatus or therapy device and/or ii) the fixation device based on the control signal to a) influence the imaging or therapy procedure and/or b) to adjust the fixation device so as change the manner in which the part is being held.

In a further aspect there is provided a method of training an initial machine learning component to obtain the pre-trained machine learning component.

In a further aspect there is provided a computer program element, which, when being executed by at least one processing unit, is adapted to cause the processing unit to perform the method as per any one of the above embodiments.

In a further aspect there is provided a computer readable medium having stored thereon the program element.

According to a further aspect there is provide a fixation device configured to hold an anatomic part of a patient during imaging with an imaging apparatus and/or during delivery of a therapy by a therapy delivery apparatus, wherein the fixation device is adjustable so as to change a pressure distribution excreted by the fixation device on at least a portion of the part.

In embodiments, the fixation device includes an adjustable contact surface urgeable into contact with the part, wherein the said contact surface shape is adjustable so as to conform to at least a shape of a portion of the part.

In embodiments of the fixation device the contact surface is formed from a plurality of contact elements individually addressable to effect a deformation or motion of same, the elements together forming the contact surface. Thus, the contact surface may be adapted in 3D.

One or more pain sensors may be used. Some or each sensor may comprise plural sensor elements arranged in a 2D array or otherwise spatially distributable around the body part of the patient.

A sensor may comprise a plurality of different sensor elements spatially distributed in relation to the object or the patient as whole. These measure sensor elements measure the same quantity at different locations. Alternatively, a plurality of different sensor of different type (some or all of which may or may not comprise different sensor elements).

Embodiments of the one or more sensor include skin conductance sensors to be brought into contact with the patient's skin. Alternatively or additionally, the sensor(s)/sensor elements may be arranged as capacitive sensors to measure pain without galvanic (that is, skin) contact.

Other sensors are also envisaged, for instance based on one or more optical cameras and image recognition to infer state of pain from facial expression or body posture.

Yet other sensor arrangements may be used instead or in combination with any of the above to measure any one or more of: heart rate, blood pressure, breathing rate or any other physiological quality correlated to state of pain.

In embodiments, one or more pain sensors may be integrated into the fixation device. There is a common housing that houses both, the one or more pain sensor and the fixation device. The housing may be configured to conform in shape or size to the body part to be imaged or treated. Alternatively, the fixation device and the pain sensor(s)/sensor elements are arranged in separate housings or there is no housing for fixation device but only a housing for the pain sensor(s). The pain sensor(s) may be arranged on the inside of the housing so they can be arranged in contact or proximal to the body part/skin to promote uniform sensor measurement conditions. A housing is however not necessary required for neither fixation device not pain sensor(s). in embodiments, the pain sensor(s) may be integrated into a wearabale such as skin sticker(s), a bandage or a piece of textile.

The proposed system may then help to optimize for a trade-off between minimum pain and low image quality versus best image quality with acceptable distributed pain.

The system performs automatic patient pain monitoring and interacts with the imaging apparatus. The interaction of the imaging support system with the imaging apparatus and/or the fixation device allows to react to a patient's condition by changing the imaging procedure and/or adjusting the fixation device.

The computing of the control signal may include, explicitly or implicitly, comparing the pain measurements against one or more pain thresholds. The pain thresholds may be used implicitly as constraints in different optimization algorithms.

The fixation device allows exerting different levels of physical pressure, and thus compression, to improve contrast sensitivity and specificity.

The proposed system allows overcoming to reduce pain and hence stress-level which in turn may result in better image quality and higher image throughput because of cooperative patient behavior. Unnecessary image retakes may be avoided or reduced.

The proposed system, method and fixation device may be used with benefit in autonomous imaging or therapy where a patient cannot easily express pain experience and point out specific pain areas to a HC professional - either because they are unable to or because there is no HC professional available.

Although main reference will be made to MRI, x-ray mammography and other imaging modalities such as ultrasound, SPECT, PET are also envisaged. Also, imaging of the human breast is a preferred embodiment herein, but the imaging of other anatomic parts is specifically envisaged herein such as brain imaging, imaging of the knee, heart imaging, etc.

The proposed system, method and fixation device may also be used with benefit in linac therapy imaging systems, such as MR linac, and many others.

The proposed system may also be used for therapy device such as the linac without imaging so that the treated body part remains stationary and pain is managed as before whilst the other quantity to monitored is now not the IQ (or not only) but the treatment quality in terms of therapy quality parameters as per treatment plan, such as dose to delivered, region to be irradiated, etc, such as in radiation therapy, in particular (but not only) intensity modulated radiation therapy (IMRT).

Definitions

"*2D*", "*3D*" as used herein denote, respectively 2-dimensional and 3-dimensional as applicable to data format or space or degrees of freedom.

In the following, the term "*user*" indicates an operator of the imaging apparatus and/or the person who wishes to acquire the images. This includes in particular healthcare professionals (e.g., radiologists, nurses, etc.). The "user" may include a radiologist or other medically qualified personnel.

The term "*patient*" will be used herein to indicate the person to be imaged.

The term "*imaging procedure*" as used herein in embodiments may indicate a mode of operation of the imaging apparatus where image data is acquired. This mode of operation includes exposing the patient to interrogating signals, such as one or more RF pulses in MRI or exposure to X-radiation, and accordingly for other imaging modalities.

BRIEF DESCRIPTION OF THE DRAWINGS

Fig. 1 shows a schematic block diagram of an imaging arrangement;
Fig. 2 shows a block diagram of an MRI imaging apparatus;
Fig. 3 shows a top view of the imaging arrangement in particular of an imaging support sub system;
Fig. 4 shows block diagrams of components of an imaging support subsystem;
Fig. 5 shows a cross sectional view of a fixation device for holding stationary an anatomic part of a patient during imaging;
Fig. 6 shows a block diagram of interfaces of the fixation device;
Fig. 7 shows elements of a machine learning component;
Fig. 8 shows a flow chart of a method of supporting imaging; and
Fig. 9 shows in more detail sub steps of a method of supporting imaging.

DETAILED DESCRIPTION OF EMBODIMENTS

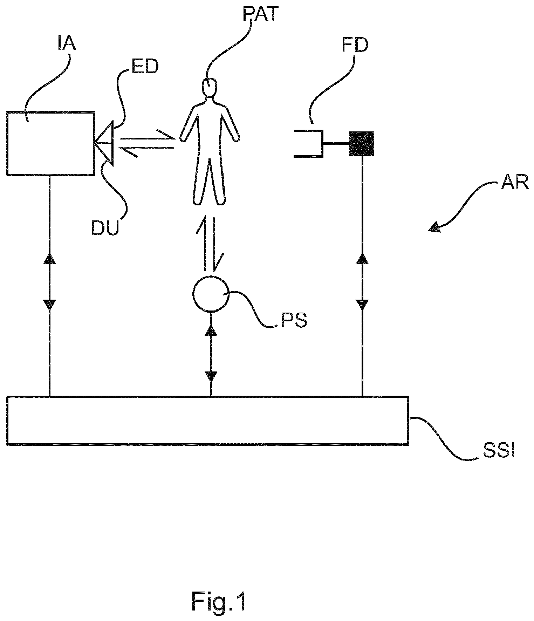

Referring first to Fig. 1, this shows a schematic block diagram of an imaging arrangement AR as envisaged herein in embodiments. The imaging arrangement AR includes an imaging apparatus IA configured to acquire medical images of a human or animal patient PAT. Different modalities are envisaged herein including, preferably MRI, but also x-ray, emission imaging such as PET or SPECT, or ultrasound imaging are all envisaged herein.

In operation, the imaging apparatus IA performs an imaging procedure. During the imaging procedure the patient PAT is exposed to an interrogating signal such as ionizing or non-ionizing radiation emitted from an optional emitter device ED. In emission imaging such as PET, there is no emitter device ED. In this case the interrogating signal is emitted by a radioactive tracer substance from within the patient, the substance having been previously administered to patient PAT.

Exposure to the interrogating signal may be restricted to a certain region of interest or anatomic part such as a human female breast, more particular the mamma region. Alternatively, whole body imaging is also envisaged. Tissue of the patient interacts with the interrogating signal. For example, in MRI, the interrogating signal is an RF signal whilst in X-ray imaging the interrogating signal is the X-ray beam, and so on for other modalities. The interaction with patient tissue results in a modified interrogation signal. The interrogating signals are detected by a detection unit DU of the imaging apparatus. For example, in MRI, the detector unit DU is an RF coil, in X-ray it is an X-ray sensitive detector, and so on for other modalities. The detection unit DU produces detector signals during the detection which may be converted by a conversion circuity into image (raw) data. Thus, the imaging procedure yields a set of image data. The image data maybe processed into medical imagery by a computing unit (referred to herein as a reconstruction processor) that runs suitable reconstruction algorithm. In this manner, imagery of a certain region of interest or anatomic part such as a human female breast may be obtained. The imagery may be displayed on a display device, stored in a memory or otherwise processed. The imagery can be used for diagnostic purposes. The imagery preferably reveals details from within the patient.

For a range of imaging modalities, such as MRI, it is important that the patient, in particular the anatomic part to be imaged, remains stationary during the acquisition of the image data in the imaging procedure. To this end, a fixation device FD is provided as part of the imaging arrangement. The fixation device is configured to fix the anatomic part of the patient and hold it still during the imaging procedure. The application mostly envisaged herein is for MRI mammography which has been found to yield excellent soft tissue resolution and is useful and partly superior to conventional x-ray based mammography.

As compared to traditional x-ray mammography, MRI imaging procedures usually last longer, in the region of tens of minutes, for example 10, 20, 30, or even 40 *mins* are not unheard of. To increase imaging quality, it is desirable to not only hold the breast still by the fixation device but also to compress the breast. The fixation device FD has thus a dual function: to hold the breast still and to produce sufficient compression to achieve good image quality. However, the compression for prolonged periods of time may cause discomfort for the patient.

To lessen the level of discomfort, but also to ensure, sufficient imaging quality at a high throughput, the imaging arrangement AR further includes one or more pain sensors PS1, PS2. These are suitably arranged in relation to the imaged patient to acquire "pain signals" that are known to be correlated to pain as experienced by the patient, due to compression of the body part such as the breast during the imaging procedure. In the following, reference will be made to "the" pain sensor, with the understanding that more than one pain sensors may be involved.

The "triad" of components, that is, the imaging apparatus IA, the one or more pain sensors PS and the fixation device FD, are suitably coupled by wireless or wired connection with a computerized system SSI configured to support operation of the imaging apparatus IA.

The imaging support system SSI, referred to herein in short as "SSI", may allow in particular autonomous imaging or therapy where the presence of a user may not be necessary at all times during the imaging operation.

In a preferred embodiment, the support system SSI is arranged in a double feedback architecture to control operation of both, the imaging apparatus during the imaging procedure and of the fixation device FD. The control operation performed by the SSI is based on the signal readings received from the one or more pain sensors PS.

The first loop, referred to herein as the pain management loop, or "PM loop", operates as follows: If the pain signal received by the pain sensor exceeds a certain threshold, or if a certain threshold is maintained over a configurable period of time, the fixation device is readjusted so that the pressure exerted on the breast is changed during the imaging procedure to lessen the pain level experienced. The readjustment is based on fixation device control signals computed by the SSI. The fixation device control signal includes fixation device control parameters that are passed on to the fixation device through a suitable interface to effect the readjustment. The fixation device control parameter(s), if applied, implements a setting of the fixation device to achieve a desired manner in which the breast is held, such as a desired (spatial) pressure distribution. The setpoint for this loop is one or more pain thresholds. In the following, if there is a reference to "the" pain threshold, it is understood that more than one such thresholds may still be used.

However, depending on how liberal the readjustment, the breast may be still allowed to move and/or the compression level to drop too low. As a result, image quality ("IQ") may be compromised. Therefore, to better negotiate pain management and IQ management objectives, there is a second feedback loop envisaged, referred to herein as the "IO loop", where the ISS controls, in particular changes, a currently used imaging procedure to ensure IQ. The IQ may be set the second setpoint for this feedback loop. The IQ may be quantified in terms of a data consistency measure, signal-to-noise ratio, contrast-to noise-ratio, or other, or combination of all the foregoing.

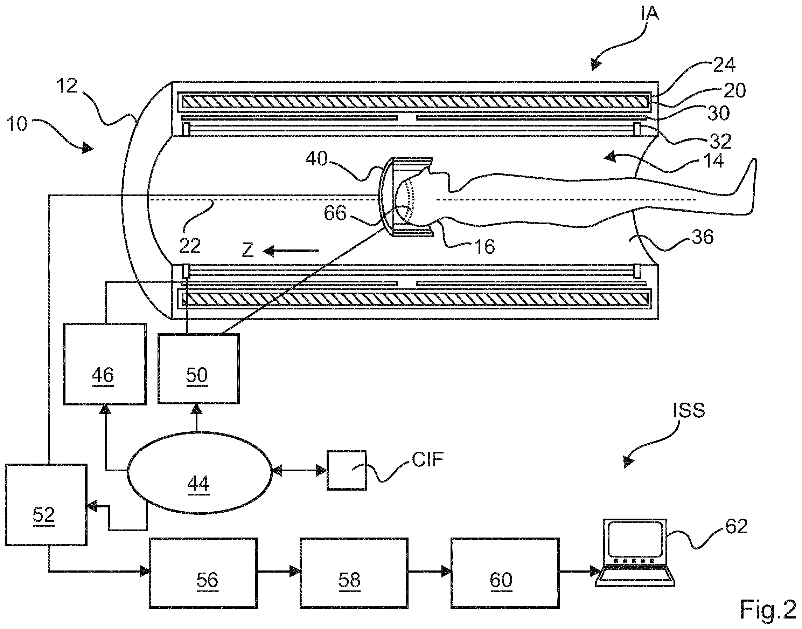

The image procedure control wielded by the SSI to enforce this second loop, may include computing by the SSI an imaging procedure control signal. The imaging procedure signal, if applied to the imager IA, implements a certain setting of imager IA. The imaging procedure control signal determines imaging control parameter(s) to change the current imaging procedure, or to suspend an ongoing imaging procedure, or to resume a previously suspended imaging procedure. The imaging procedure determines how the patient is imaged, on which more further below at Fig. 2, where an imaging procedure will be explained in more detail with reference to the exemplary embodiment of MRI.

In the double feedback architecture of the system SSI, pain signal as acquired by pain sensor PS are still received at a suitable sampling rate during the readjustment of the fixation device FD. Once a more comfortable adjustment has been achieved, which is evidenced by the received pain signals dropping (again) below the pain level threshold, the imaging procedure may proceed from the point where it was suspended.

Once the imaging procedure has terminated, the support system SSI may evaluate whether the image date acquired during the, possibly interrupted, imaging procedure still allows reconstruction of imagery at a sufficient image quality. If the SSI decides that the image quality cannot be maintained or increased, the whole or part of the imaging procedure is re-run. On the other hand, if it is decided that the image quality can be maintained, that is, that the one or more interruptions during the imaging procedure had only a negligible impact on the imaging quality, the acquired image data may then be passed on to the re-constructor to reconstruct the image data into imagery.

The proposed imaging arrangement AR, thanks to the feedback architecture of the support system SSI, may hence help to reduce the pain level experienced by the patient and may still help to attain preset image quality at an increased imaging throughput as unnecessary re-imaging can be avoided.

In the above and the following, for ease or presentation, in the pain thresholding it is assumed that a drop under the pain threshold represents a state of no pain whilst a reading of the pain signal above the threshold indicates a state of pain. This assumption is however by convention only and not limiting and furthermore may depend on any one or more of: i) the type of sensor used, ii) the encoding of the pain measurements and iii) the logic used in the SSI. There are set-ups envisaged where the converse is true, that is, where a numeric drop under the threshold is indicative of pain and a reading above the threshold is indicative of no pain. Both possibilities are envisaged herein.

In embodiments, the pain threshold maybe set such that a measured pain sensor value below a certain level still corresponds to some (but tolerable) remaining pain while a pain measurement above the threshold may be classified as too much pain. This thresholding could thus be personalized (e.g. in a "dry-run" before imaging) resulting in improved imaging outcome.

Pain as such is experienceable only by the individual herself. However, if the patient experiences pain, the body presents with instances of reactions. The reactions may be captured through a number of measurable quantities. The pain sensors PS 1,PS2 as envisaged herein are configured to measure one or more physiological or physical quantities that are known to be correlated with states of pain experience.

A number of different type of sensors may be used to measure different such quantities. Sensor of this different type may be used herein in combination. Some or all of the measured quantities may be combined into a combined pain score by averaging, or weighted averaging etc, and it is this combined pain score that is compared against the pain threshold to conclude that pain is experienced by the patient. Alternatively, each pain measurement may be evaluated against different thresholds and a state of pain is concluded by a logic of the support system SSI (to be described more fully below), only if a certain number of said measurements exceed (or, as the case may be, drop below) their respective threshold.

State of pain may be said to have two aspects: a magnitude aspect and a temporal aspect. The magnitude aspect may be accounted for by way of any of the above measurement thresholds. In order to account for the temporal aspect, an additional temporal thresholding may be performed based on how long a measurement exceeds or drops below the threshold. A timing circuitry may be used and state of pain is concluded only if the measurements exceed (or drop below) the threshold for a pre-set period of time.

In the following, if reference is made to "the pain sensor" or "the pain threshold", this is for brevity only and does not exclude references to the case of multiple thresholds and/or multiple sensors (possibly of different type).

Reference is now made to Fig. 2, which shows an MRI embodiment of the imaging apparatus 10 as envisaged for use in the proposed imaging arrangement AR of Fig. 1. Fig. 2 illustrates an MRI imaging procedure for brain imaging by using an optional local RF coil 40 arranged around the patient's head. However, it will be understood that similar set of such local receiver RF coils may be used for other anatomies of interests, suitably formed to correspond to the geometry of the anatomy of interest. For example, in breast imaging a similar localized set of coils may be used arranged around the patient's breast suitably formed and sized.

With continued reference to Fig. 2, a magnetic resonance imaging scanner 10 includes a housing 12 defining a generally cylindrical scanner bore 14 inside of which an associated imaging subject 16 is disposed. Main magnetic field coils 20 are disposed inside the housing 12. The main magnetic field coils 20 are shown simplified to a generally solenoidal configuration to produce a main Bo magnetic field directed along a Z-direction lying parallel to a central axis 22 of the scanner bore 14. The main magnetic field coils 20 are typically superconducting coils disposed inside in cryo-shrouding 24, although resistive main magnets can also be used. Particularly at higher field strengths and therefore higher frequencies, superconducting magnets are preferred.

The housing 12 may also house or support magnetic field gradient coils 30 for selectively producing magnetic field gradients along the Z-direction, along in-plane directions transverse to the Z-direction (such as along Cartesian X-and Y-directions), or along other selected directions. The housing 12 also houses or supports radio frequency head or body coils 32 for selectively exciting and/or detecting magnetic resonances.

Although birdcage coils are common at 128 MHz and below, other coils besides a birdcage coil can be used as a volume transmit coil, such as a transverse electromagnetic (TEM) coil, a phased coil array, or any other type of radio frequency coil. The housing 12 typically includes a cosmetic inner liner 36 defining the scanner bore 14. A bed BD may be arranged inside to bore for the patient to lie on during imaging. Instead of or in addition to the, in general, stationary radio frequency coil 32, a local radio frequency transmit and/or transmit/receive coil such as the illustrated head coil 40 (of TEM or other type) can be employed. The illustrated local radio frequency coils 40 is optional. Preferably it is mobile and may be used if no whole body scan is done, but only a local scan of an anatomy of interest such as of the head or breast is desired. The mobile coil 40, such as the TEM, may be moved close to the anatomy to be imaged to at least partly surround the local anatomy.

The main magnetic field coils 20 produce a main magnetic field B0 in a Z-direction which is preferably at least 3.0T, and more preferably greater than 3.0T, such as 7.0T or higher. A magnetic resonance imaging controller 44 operates magnet controllers 46 to selectively energize the magnetic field gradient coils 30 and operates a radio frequency transmitter 50 coupled to one or more of the radio frequency coils 32,40 to selectively energize the radio frequency coil or coils 32,40. By selectively operating the magnetic field gradient coils 30 and the one or more radio frequency coils 32,40, magnetic resonance is generated and spatially encoded in at least a portion of a selected region of interest of the imaging subject 16. The magnetic resonance imaging controller 44 operates a radio frequency receiver 52 coupled to one or more of the radio frequency coils 32,40 to receive image data in form of magnetic resonance k-space data samples that are stored in a k-space memory 56. The image data may be stored in any suitable format.

A reconstruction processor 58 applies a suitable reconstruction algorithm such as a Fourier transform reconstruction algorithm to reconstruct the k-space samples into a reconstructed image including at least a portion of the region of interest of the imaging subject. The reconstructed image is stored in an image memory 60, displayed on a user interface 62, stored in non-volatile memory, transmitted over a local intranet or the Internet, or otherwise viewed, stored, manipulated, or so forth. The user interface 62 can also enable a radiologist, technician, or other operator of the magnetic resonance imaging scanner 10 to communicate with the magnetic resonance imaging controller 44 to select, modify, and execute magnetic resonance imaging sequences.

In one example imaging sequence, the TEM head coil 40 is energized by a radio frequency excitation to excite magnetic resonances of $^1H$ in the region of interest of the imaging subject 16. For an example main magnetic field $B_0$=7.0T, the corresponding magnetic resonance frequency is $f_{res}=\gamma B_0$=298 MHz, where $\gamma \approx$ 42.58 MHz/T is a proton gyrometric ratio. At this frequency, resonance signals have a wavelength in air of about one meter, but in the human head with an average permittivity of $\varepsilon_r$ = 40 the wavelength is $\gamma$ about 16cm, which is smaller than many typical imaging regions of interest. For other body parts to be imaged, such as the breast BR, the above numerical examples may need to be adapted.

During the radio frequency excitation, the magnetic field gradient coils 30 produce a slice-selective magnetic field gradient along the Z-direction to select an axial slice or slab 66 that meets the resonance condition for excitation. Rather than selecting an axial slice, a coronal, sagittal, or otherwise-oriented slice can be selected using a suitably directed magnetic field gradient applied during the radio frequency excitation.

The resonance signals from the selected slice are read out using a sequence of phase encode gradients applied along an in-slice phase encode direction, and readout gradients applied along an in-slice readout direction transverse to the phase-encode direction. In another suitable readout, in-slice magnetic field gradients are applied along selected angular directions transverse to the Z-direction and spanning 180° or 360° of angular views. The slice or slab imaging sequence is repeated for a plurality of adjacent or spaced-apart slices to produce imaging data that is suitably reconstructed by the reconstruction processor 58 into volumetric or multi-slice reconstructed image data. The described imaging is only an example. Those skilled in the art can readily modify the described multi-slice sequence or construct other multi-slice imaging sequences that include features such as spin refocusing, an echo planar readout, contrast enhancement or selection mechanisms such as inversion recovery pre-pulses or the like, and so forth.

The readout and/or imaging pulse sequences mentioned above are defined by the above mentioned imaging control parameters applied by the imaging controller 44 to the coils and/or other components of the scanner 10. The image control parameters hence define the imaging procedure. The imaging control parameters in particular describe how the imaging or "scan" is to be performed, in particular the size of the field-of-view, which slice is to be scanned and/or which imaging or readout pulse sequence is to be used, how to influence the magnetization, how the image contrast is defined etc.

The nature of the image control parameters may differ for other imaging modalities. In X-ray imaging for example, the control parameters include quantities such as tube voltage, cathode amperage, exposure time, distance, projection parameters etc. Other imaging modalities envisaged include ultrasound or imaging in combination with radiation therapy (MR-linac).

For any imaging modality, the term "imaging procedure" is to include in particular a reference to all or a subset of the control parameters. A change of an imaging procedure includes in particular a change of one, more than one, or all imaging control parameters. "Imaging protocols" are rules that prescribe one or more imaging procedures for particular anatomy of interest and/or purpose of the imaging. The terms "scan" and "imaging" may be used interchangeably herein.

The magnetic resonance imaging controller 44 is communicatively coupled in wired or wireless connection through interface CFF with the imaging support system SSI to receive data (such as new control parameters) from to the SSI and/or to provide data to the SSI.

It will be understood that the components of the above described MRI scanner 10 in Fig. 2 is merely according to one embodiment. Any other type MRI scanner is also envisaged herein in alternative embodiments. Combined MRI and PET/SPECT "tandem" imagers are also envisaged. In PET/SPECT, there is may be an additional memory to store image data as nuclear incident data, for example in list mode or any other format.

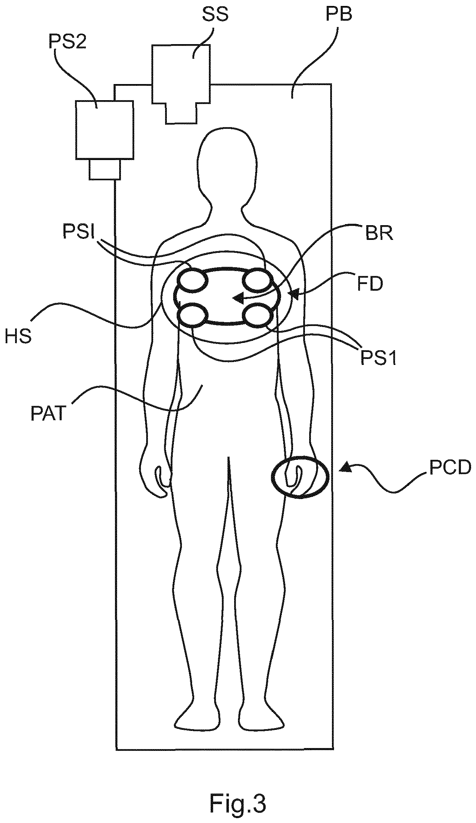

Referring now to Fig. 3, this shows a top view of the patient PAT during the imaging procedure, with components of the imaging apparatus IA partly cut away for ease of presentation. The patient may rest on a patient bed PB arranged in the imager IA, such as in the bore of the MRI imager of Fig 2. Bed PB is optional as other embodiments are also envisaged where the patient PAT is standing during imaging such as during a chest x-ray or during conventional X-ray based mammography. The pain sensors (indicated by four circles) PS1 maybe integrated into the fixation device FD as schematically shown in Fig. 3. The fixation device operates to hold the patient's PAT breast BR stationary and to compress same to ensure image quality ("IQ").

Additional one or more pain sensors in the form of an optical camera PS2 may also be used. In other embodiments however, additional safety sensors SS are used such as an optical or other sensor camera that does not necessarily form part of the pain sensor system but is arranged to enforce safety requirements. The function of such as safety sensor SS is to detect the patient, estimate the distance between patient and sensor and to estimate a safety value. The safety sensor SS may be used to monitor that the patient is correctly positioned inside the scanner IA, and/or, in MRI, that the coils are in place and/or that the pain sensors PS1,PS2 are placed correctly. The safety sensor SS may also be integrated in coil 40 or the fixation device FD. The optional safety sensor is configured to monitor and feedback-control the correct position or deviations therefrom of patient PAT during the imaging procedure.

Arrangement AR may further include a user operable user interface PCD such as switch with which the user PAT herself can control the fixation device to ease compression levels and hence pain. The user is preferably able to override the SSI for immediate pain relieve. Preferably imaging procedure is interrupted if the user requests through the interface PCD readjustment of the fixation device FD.

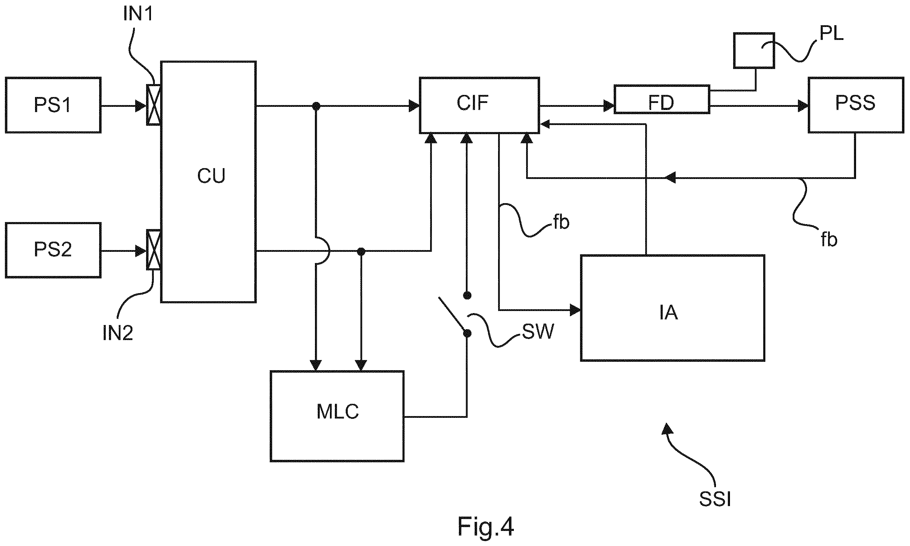

Referring now to Fig. 4, this shows in more detail a block diagram of the proposed imaging support system SSI. The support system SSI maybe arranged wholly or partly in software on a general purpose or dedicated computing device. In embodiments, the SSI may be integrated into a workstation associated with the imager AI (or with a group of such imagers). Instead or in addition, the SSI maybe arranged partly or wholly as hardware in the form of suitably programmed microcontrollers such as FPGA's, ASICS or others, with suitable interface equipment. The SSI may be fully or partly integrated into the imaging apparatus IA. The SSI may be integrated into an operator console or image control unit 44 of the imaging apparatus IA.

The block diagram in Fig. 4 illustrates in particular the one or more, preferably two or more, feedback loops *fb* as envisaged herein, including the PM loop and the IQ loop. The SSI may include a control unit CU. Operation of the two loops are administered by the control unit CU through a suitable control interface CIF. Control signals to the fixation device and imager as passed on trough this interface and feedback is received through the interface IFC and passed back on to the logic of controller CU. The data processing in the controller CU is preferably in realtime and/or simultaneously, parallel.

Preferably, the two loops are linked and may be conceptualized by operation in a parameter space or phase space, $S = (x_{IQ}, x_{FD}, x_{IA}, x_p) \subset \mathbb{R}^k$, in general of dimension at least 3 but generally higher.

$x_{IQ}$ is an image quality parameter which may be expressed in terms of signal to noise ratio, contrast to noise ratio or a combination of these.

$x_{IQ}$ may instead or in addition relate to consistency measure that quantifies whether image data has been compromised by intra-imaging motion or compression change.

$x_{FD}$, $x_{IA}$ relate to the FD control parameter and the imager control parameters, respectively, *xp* are the pain measurements.

The operation of the control unit, that is, the computing of the control signals, may be represented as respective constrained mappings F1, F2 in this space S. This may be formalized as:

$$F1: x_p \rightarrow x_{FD} \text{ such that } x_p < T_p \text{ and} \tag{1}$$

$$F2: x_{FD} \rightarrow x_{IA}, \text{ such that } x_{IQ} > T_{IQ}, \tag{2}$$

with $T_p$ and $T_{IQ}$ denoting the pain threshold and the IO threshold, respectively. The threshold may be user definable or hard-coded.

Note that it may not always be possible to reconcile both, F1 and F2, and a deadlock situation may occur. Preferably, the pain constraint takes preference. If it is not possible to achieve $x_p < T_p$ and $x_{IQ} > T_{IQ}$, the imaging may be aborted. An alert signal such as a flash light, pager/email/text message, alert sound etc. may be issued through a suitable transducer to alert the user of the deadlock situation.

The logic implemented in the control unit CU approximates mapping *F1, F2*. In embodiment, the fixation function F1 may be implemented by a random generator to generate by random different readjustments of the fixation device in an attempt to lower the pain level under the threshold. Preferably, the mapping F1 is implemented deterministically.

Specifically, the mappings F1, F2 may be estimated conventionally by tables, such as LUTs or interpolation from known functional expressions or by statistical techniques. In a preferred embodiment however, one or both of the mapping *F1,F2* are implemented by a machine learning component MLC. The machine learning control unit MLC may be coupled to the CU in parallel and has as input the pain measurement data as supplied by the one or more sensors PS1, PS2. A switch SW may be provided that couples the MLC to the CU or direct to the control interface CIF. The control interface CIF provides the control signal for the fixation device FD and/or the imager IA.

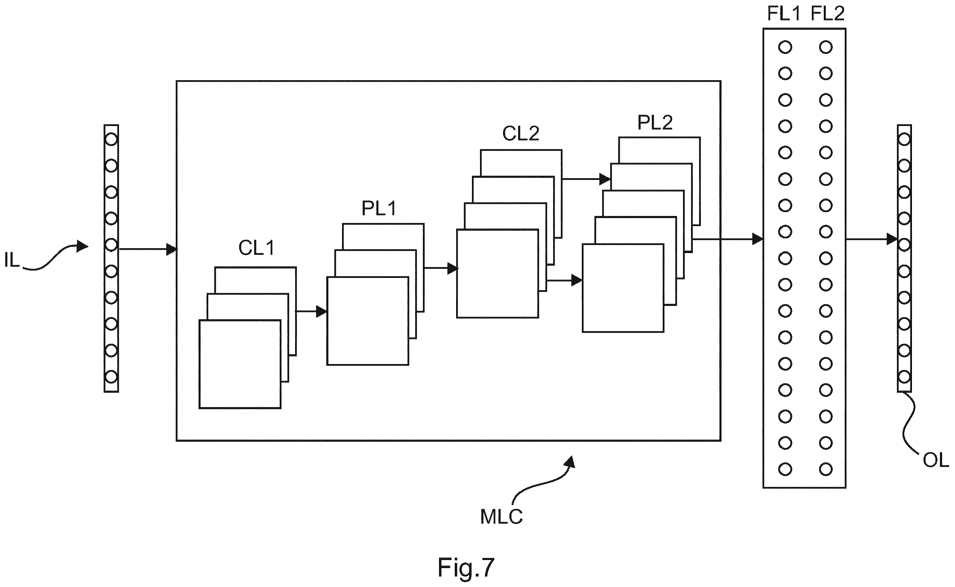

The machine learning component may be external to the control unit or the machine learning component may be incorporated in the logic of the control unit CU. The machine learning component MLC implements a machine learning algorithm. The MLC has been pre-trained on suitable historical data. A neural network embodiment of the MLC will be explained later in more detail below at Fig. 7.

In more detail, and with continued reference to Fig. 4, the CU computes, based on the pain measurements received from sensors PS1, PS2 a target control parameter for fixation device FD. Application of this control parameter may result in a different compression on the breast which may or may not be good enough to maintain a desired IQ. The CU computes based on the new fixation device control parameter, an imaging control parameter that is capable of achieving the desired IQ target, given the expected compression/motion caused by the newly computed fixation device adjustment. If both, pain level and IQ comply with the thresholds, the control signals are passed on to the imager IA and the fixation device FD through control interface CIF. At the interfaces the desired control parameters as computed by the CU are compared with current values. Based on the comparison, as set of respective control commands, for the fixation device and the imager IA, are selected. The control commands are selected to achieve the target. The imaging control commands are then used to instruct the imager control module 44. The control module then applies possibly new imaging control parameters to relevant time imaging components such as to the magnetic or RF coils in the MRI to change the imaging procedure and to ensure desired image quality. The instructions to the imager IA, based on the computed imaging control parameters, may include instructions to interrupt an ongoing imaging procedure or to resume a previously interrupted imaging procedure or to change the RF pulse sequence and/or a new definition of the spatial slice through the patient's breast or other anatomic part to be imaged.

In a similar manner, the new fixation device control parameters are applied to the fixation device to effect a new setting and to achieve a different pressure distribution on the breast.

The imaging procedure is not necessarily interrupted when readjusting the fixation device FD, provided the required TQ does not drop. Certain fixation device readjustments may be allowed to be performed during the imaging procedure. Fixation device adjustments applied during an ongoing imaging procedure are likely those that incur only a marginal breast compression change, which sometimes nevertheless can cause great pain relief.

It will be understood that if the machine learning component MLC (referred to hereinafter briefly as the "MLC") is used by the control unit CU, there may not necessarily be a need for an explicit thresholding check as mentioned above at functions *F1,F2* at eqs (1) and (2), as the MLC has been trained to inherently respect the pain level and IQ thresholds. However, in embodiments, even if the MLC is used, there may still be any iterative (that is, repeated) thresholding check in the PM and/or IQ loop as an extra safety and quality control layer. If non-ML algorithms are used, it is preferable to have iterative threshold checking performed in the PM and/or IQ loop whilst readjusting the FD and/or changing the imaging procedure. The iterative thresholding is executed as a suitable sampling frequency which is either hard-set or is user adjustable.

Preferably, pain measurements supplied by the pain sensors PS1, PS2 are digitized and any signal processing such as filtering, etc. is preferably performed in the digital domain. The control interface CIF has a digital or analog output to steer the fixation device FD.

Wired communication links within the SSI and between the SSI and the fixation device and/or imager IA are preferably EMC compatible with MRI electromagnetic RF fields. In embodiments, optical communication maybe used. For example, wired links between sensors PS1, PS2 and controller CU are arranged as optical cables.

Imaging support system SSI may further include a pain locator component PL that operates the fixation device FD in a pain locator mode as will be explained in more detail below.

Although in the embodiment of Fig. 4 a single control unit CU is shown to receive measurements from all pain sensors, this may not necessarily be so in all embodiments. In other words, in alternative embodiments different control units (CU1, CU2 etc.) may be provided to process respective pain level measurements from respective pain sensors PS1, PS2.

If the image procedure was interrupted once or more, the image data currently resident in image data memory 56 may comprise two or more runs of contiguous image data interrupted once or more in time. The run of image data may be evaluated for consistency using a consistency measure to ensure the intra-imaging movements or compression changes possibly incurred during the fixation device readjustments will not compromise image quality after reconstruction. If there is no inconsistency, the image data may be passed on from memory 56 to the reconstructor 58 to reconstruct the imagery. If there is inconsistency, some image data runs may still be retained in memory 56 and only the actually corrupted runs of image data are re-acquired. During the requisition the SSI operates as above described. Once re-acquired image data is found to have sufficient IQ, the reacquired image data runs together with the retained runs from the previous imaging procedures may be passed on to the reconstructor 58. The consistency check may be performed by the control unit CU or by a separate IQ checker module (not shown).

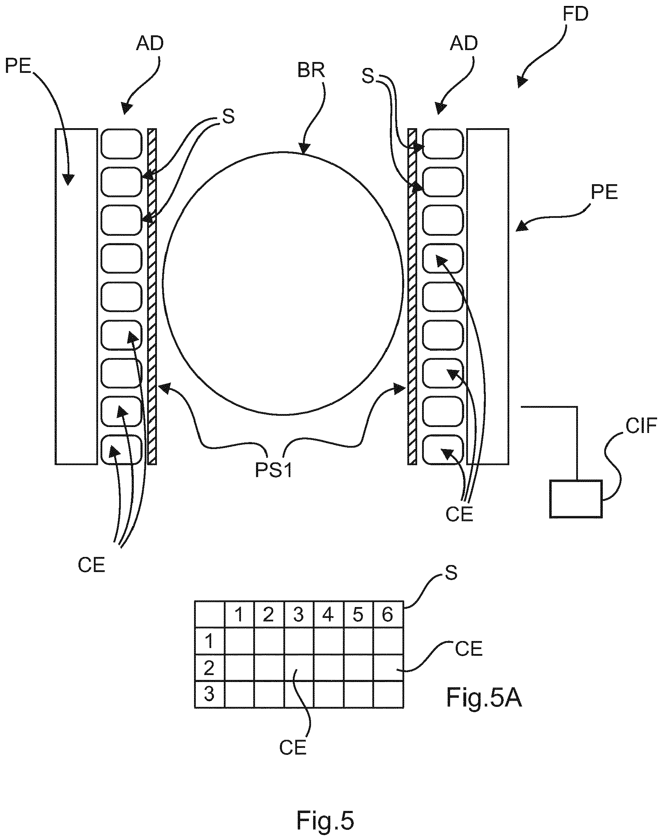

The re-adjustment of the pressure distribution may be implemented efficiently by having the fixation device FD equipped with an adaptation surface S adjustable in 3D. To explain this in more detail, reference is now made to Fig. 5 which shows, in a cross section, an embodiment of the fixation device FD as envisaged herein, preferably (but not necessarily) for fixation of a human breast BR. The following principle of operation and structure may be readily adapted to other anatomies of interest, such as knee, head, arm or other.

In this or similar embodiments, the fixation device FD comprises two pressure actuators PE arrangeable, in use, on both sides of the breast BR. In the embodiment the pressure actuators move in opposed relationship towards each other, and hence towards the breast, to engage the breast and effect a squeeze action on same. The fixation device, and in particular the pressure actuators PE, may be arranged in a suitable housing HS with guiding elements to guide the motion of the actuators. A further function of the housing HS is to carry the one or more pain sensors, in particular in embodiments where the pain sensor(s) are integrated in the fixation device. Specifically, in embodiments, the fixation device FD housing may be arranged so as to at least partly conforms to the shape of the relevant body part, such as the breast, where the housing is cup-shaped for instance. Having such housings that conforms in shape and/or size to the relevant body part allows ensuring comparable sensor conditions at most or all regions of the skin / body interface. This is advantageous if the pain is measured via several pressure sensors/sensor elements arranged in the housing. Different housings may be provided as a set in different sizes such as small/medium/large to cater for different breast sizes for instance. The housing(s) may be manufactured via 3D printing or other additive manufacturing techniques but subtractive manufacturing may be used instead. Such fixation device housings may be provided for body parts other than the breast, such as breast, arm, leg, ankle, head or other parts of the body which are clinically relevant for imaging or therapy.

The above described housing HS may also be provided separate from the fixation device when the sensors PS1,PS2 are not integrated with the fixation device FD. In this embodiment the housing HS is a dedicated pain sensor housing, not integrated with the fixation device FD. As before, the dedicated housing is configured to carry the sensors and to provide the above mentioned comparable sensor conditions, particularly when a plurality of distributed pain sensors/sensor elements is used. The one or more sensors maybe attached in the inside of the housing so as to fully or partly surround the body part when the body part is inserted into the housing.

Although the fixation device FD and or the housing is mainly envisaged for imaging, either may be used instead or in addition for therapy and/or in combination with other devices and treatment modalities such as elastography or local hyperthermia. For example, in local hyperthermia, the local pain is not induced due to mechanical stress but by temperature exposure.

The pressure actuators PE allow exertion of a pre-defined amount of pressure on the breast to achieve the desired compression so to ensure IQ. However, in order to be able to better re-distribute the pressure across the surface of the breast, the fixation device FD further comprises a shape adaption component AD. In embodiments, the shape adaption component AD comprises two sets of contact elements CE, one set arranged on each pressure actuator PE as shown in Fig. 5. The contact elements, or pads, are each individually addressable and actuatable. For instance, each contact element CE may be moveable towards the breast independently from the other contact element(s) CE. Other forms of independent actuation may include effecting shape changes, such as through inflation or other. The actuation of the pressure actuators and/or the individual contact elements CE may be driven by pneumatic, hydraulic, piezo-electrically, mechanical gearing or any other arrangement.

Portions of the contact elements CE proximal to the breast form the contact surface S. The shape of contact surface in 3D may be adjusted by actuating the contact elements individually. A wide range of different surface shapes can be realized in this manner to conform to the breast shape of a given patient whilst exerting the overall pressure. Thanks to manifold shape adaptation options, the pressure distribution can be changed in fine gradations to alleviate pain in a locally targeted manner. The pressure actuators operate to exert an overall pressure and the pressure fine-tuning is done by controlling the contact elements.

Inset Fig. 5A shows a frontal view of the contact surface that is urged into contact with the breast. Preferably the contact elements CE of each set are arranged in a respective array, for example a 6 x 3 array to form 18 contact elements, each individually addressable as explained. In this manner the contact surface S can be changed in 3D. Similar embodiments are also envisaged where each contact element is arranged as a strip extending into the drawing plane of Fig. 5. This, however, is less preferable as this embodiment allows less degrees of freedom and hence changing the surface only in 2D.

In each of the above described embodiments, the position, shape, inflation or other actuation of each contact element CE is addressable from the control unit CU through control interface CIF. The fixation device is monitored by a further sensor PSS, which measures the actuation, e.g. progression, of each individual contact elements CE. An additional feedback signal tells the control interface CIF about the status and mechanical progression of each element CE.

Although Fig. 5 shows a two-sided embodiment where the left and right pressure actuators PE are each actuatable, this may not be necessarily so in all embodiments. Alternatively, there is only one pressure actuator which actuates against a stationary component such as a fixed compression plate. As a further variant, there may only be a single adaptation component AE arranged on only one of the pressure actuators or compression plate.

In a preferred embodiment, one of the pain sensors PS1 may be integrated into the fixation device FD as shown in Fig 5. Preferably the pain sensor is coupled to the proximal portions of the contact element CE.

Yet more preferable, in embodiments where the pressure sensor PS1 is so integrated, the pressure sensor PS1 is arranged as one or two respective arrays of discrete sensor elements. The sensor elements are in spatial registry with the respective array of contact elements CE as shown in Fig. 5 in side view. Only one dimension of the pain sensor element array and contact elements CE is shown, in different rows, whilst the columns of the matrix run into the drawing plane of Fig. 5. In this manner the pressure sensor is capable of providing a pain map, that is, a spatially (2D) resolved indication of pain loci, that is, locations where on the breast the pain originates from. In this embodiment, but also in other embodiments, the pressure sensor array may be arranged as galvanic contact elements configured to measure galvanic skin conductance (GSC) of the patient. Each pain sensor element in the matrix may comprise a pair of electrodes, optionally coupled to a high-impedance amplifier attached to a part of the patients' body. Typically, each electrode has a size of about 1 cm$^2$ and each pair are separated by 1-5cm.

The sensor or array of sensor is located close, but not necessarily in contact, to the skin surface. This proximity may be achieved when having the sensor or sensor array arranged inside the body-part-shape-conforming housing. The sensor elements of the array may be arranged in capacitive technology that does not require skin contact. In this capacitive embodiment, the sensor elements are arranged in galvanic isolation from the skin. In addition or instead, the sensor or sensor elements are electromagnetic or optical. Temperature sensor elements are also envisaged for pain measurement purposes.

The pain measurements can be taken with high accuracy when the sensor elements are urged into contact with the patient's skin whilst the breast is being held stationary and under compression by compression or pressure actuator(s) PE.

Measurement of the GSC does not necessarily imply that the above described sensor element array is used. In simpler embodiments, the pain sensor is arranged as a single pair or electrodes or as more than one such pairs suitably distributed and to be brought into contact with the patent's skin, not necessarily where the anatomy of interest is located.

GSC-based pain sensors are merely one embodiment of pain sensors configured to pick up vital signs correlated with pain. Other vital sign sensors PS are also envisaged, so long as the respective vital sign is known to respond rapidly to acute pain. Indeed, GSC has the advantage that a rapid and large increase of skin conductance is recorded around 1 second after an acute pain event, whereby corrective action can be taken before the patient suffers a prolonged periods of pain. Further embodiments of vital sign based pain sensor includes one or more optical cameras for video-based physiology monitoring. Thus detection of changing breathing rate and/or heat rate can also indicate a presence of pain.

In addition or instead, integrated motion sensors may also be used as pain sensors as increased patient motion may indicate that the patient experiences pains. Envisaged motion sensors for pain measurement include any one of piezo elements, RF-impedance based sensors, and radar-based sensor, and others. RF-impedance based sensors may include "low"-frequency technology, e.g. by using the MRI-coils as transmitter/receiver. Subject PAT motion can be detected by changing signal transmission and/or reception. In addition or instead, "high" frequency could be based on radar measurements. In radar-based methods or similar, time-of-flight may be applied, similar to technologies for distance detections in automobiles. Motion can also locally be detected by having one or more of the pain sensor arranged as an optical cameras or other optical sensor measuring the surface of the skin.

Integration of the pain sensor PI into the fixation device FD is preferred but this is not the only embodiment envisaged herein. For instance, in addition or instead of having the pain sensor PS1 integrated in the fixation device FD, the pain sensor(s) may be integrated into the local RF coil 40. In yet other arrangement, the pain sensor is a separate stand-alone piece of equipment with a wearable component attachable to the anatomy of interest such as a bandage component or other textile item in which the sensors PS1 are included.

Other embodiments for the pain sensor system PS1, PS2 includes an optical camera device for recording a facial expression and/or body posture. An image recognition processor may then analyze the imagery captured by the camera to conclude whether or not the patient is in pain. States of facial distortion or cramps or spasm of the body indicative of pain maybe recognized in this way.

Further embodiments include audio-based pain sensor systems that include one or more microphones arranged in or at the imager IA communicatively coupled to a speech recognition processor. Sound recorded by the microphone as uttered by the patient during imaging may be picked up and analyzed by the speech recognition processor. The speech recognition processor may be adjusted to recognize in particular interjections such as exclamations or curses commonly associated with pain experience such as "ouch", etc. The Speech recognition processor may be able to handle such pain-indicative interjections in multiple languages. All or any sub-set of the above described pain sensor embodiments may be used in any combination to produce multiple pain measurement of different type which may then be consolidated in to single pain score to be compared against the threshold. Evaluation of multiple such pain measurement against multiple pain threshold are also envisaged to arrive at decision where or not there is a pain event, that is, where the patient is taken to experience pain.

The matrix arrangement of sensor elements may be of particular benefit when the SSI is operated in pain locator mode though pain localizer module PL. The pain-map may be used to optimize the fixation device adjustment for pressure direction and pressure amount accordingly.

Pain qualia, that is, the way pain is experienced by the individual, is often not only a function of the pressure exerted but also the length of time and the location where on the breast circumference the pressure applied. Certain portions of the breast may be more sensitive than other parts and this may be highly individual. The pain localizer allows finding the location of the pain source. The fixation device may then be readjusted so that pressure is eased at the pain locus, and instead is increased elsewhere, preferably by the same amount, to so maintain the overall compression, and hence IQ, and still achieve pain relief. Equally, a sensitive pain source may be temporarily relieved so that pain levels drop, instead increasing pressure elsewhere. After a certain pain relief period, which may be user adjustable and timed through a timer function of the control unit CU, the pressure is increased again at the pain locus until pain level builds up again, and so forth, thus taking advantage of the time dependency of pain.

Turning now in more detail to the pain location functionality briefly mentioned above at Fig. 3, this allows finding a pain locus (there may be more than one) and is envisaged herein through operation of the pain locator PL.

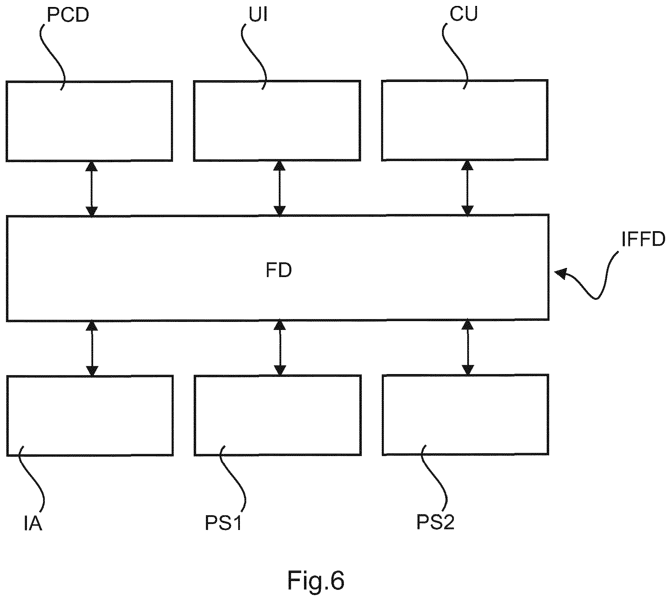

The pain locator PL controls the control unit CU to have it operate in a pain locator mode which is now explained in more detail. Pain locator PL may be included as a component (e.g., software routine) or may be arranged as a standalone control component as indicated in Fig. 2. Operation in this mode may be requested by the patient or user interfaces mentioned above in Fig. 6. Alternatively, this mode may be entered into automatically after a pre-set number of attempts of finding an acceptable fixation device readjustment failed.

It is of note that, given the above-mentioned spatial pain sensitivity variance, a point where the highest pressure is applied by the fixation device may not necessarily be the pain locus.

In order to address this and other concerns around pain, operation in pain locator mode may include "pressure sampling" the breast or a part thereof (such as the portion around the mamma), to find the pain locus.

The fixation device is controlled by the CU when delivering this mode. Such a localization mode may involve a sequential spatial modification of the pressure application using the adaption AC device of the fixation device FD. For example, the individual contact elements CE are controlled to execute, in spatial and temporal concert, a series of small actuations such as small inflations or deflations or movements, depending on the particular implementation. These actuations form a spatially and/or temporally varying pressure profile wave signal, including triangular sweeps or other profiles. The pressure wave signal delivers different pressures at different points at different time whilst the pain level is being monitored by the pain sensors. Different pain signals at different times are thus associable with different locations on the breast. An acute pain signal may then be generated by the pain sensor PS when pressure wave affects the actual pain locus. The pain locus can thus be localized by monitoring the pain signals for peaks and the corresponding location found is logged. The user may then be informed through suitably alert or messaging signals with the instruction of how to adjust relevant portion of the fixation device. Preferably, the adaptive device AD is adapted so that even a normal motion of the body part in the fixture does not induce acute pain. Alternatively, the system SSI readjusts the fixation device automatically to find a setting where the IQ can be maintained and pressure on the pain locus is reduced.

Reference is now made to block diagram in Fig. 6 which shows in schematic fashion additional interactions envisaged herein between the fixation device FD and other components of the imaging support system SSI.

Optionally, there is the patient control interface PCD briefly mentioned above for self-determined pain relief.

The patient herself is capable of controlling the level of compression and/or pressure distribution exerted by the fixation device. The fixation device settings requested through the patient control interface PCD preferably override any settings proposed or applied by the control unit CU.

Optionally, there is a user interface UI for the user of the imaging apparatus to control the fixation device in case the patient is unable to do so. If user is present (or can be called), they can take corrective action with the fixation device to re-adjust the pressure to avoid the pain once alerted by the pain sensor. However, in a more autonomous setting, as envisaged herein in embodiments, where users are not easily available - or alternatively if the patient can only communicate with difficulty - proposed system operates through interaction with the imager and the fixation device to re-adjust the pressure and adapt the imaging procedure as required, as explained above.

Referring now to Fig. 7, this shows in more detail a machine learning component setup in a CNN architecture. Preferably, however, the above mentioned relationships, in particular function *F1* and *F2,* may be encoded by a suitable trained machine learning component. The machine learning component may be realized as neural-network ("NN"), in particular a convolutional neuro-network ("CNN") but may be arranged instead as a support vector machine, linear regression algorithms or otherwise.

Main reference will be made however, to a NN setup although this again is not at the exclusion of the other mentioned embodiments.

With continued reference, Fig. 7 shows schematically an MLC architecture in CNN layout as envisaged herein in embodiments.

The CNN is operable in two modes: "training mode" and "deployment mode". In training mode, an initial model of the CNN is trained based on a set of training data to produce a trained CNN model. In deployment mode, the pre-trained CNN model is fed with non-training, new data, to operate during normal use of the SSI. The training mode may be a one-off operation or this is continued in repeated training phases to enhance performance. All that has been said so far in relation to the two modes is applicable to any kind of machine learning algorithms and is not restricted to CNNs or, for that matter, NNs.

The CNN comprises a set of interconnected nodes organized in layers. The CNN includes an output layer OL and an input layer IL. The CNN has preferably a deep learning architecture, that is, in between the OL and IL there is at least one, preferably two or more, hidden layers. Hidden layers may include one or more convolutional layers CL1, CL2 ("CL") and/or one or more pooling layers PL1, PL2 ("PL") and/or one or more fully connected layer FL1, FL2 ("FL"). CLs are not fully connected and/or connections from CL to a next layer may vary but are in generally fixed in FLs.

Nodes are associated with numbers, called "weights", that represent how the node responds to input from earlier nodes in a preceding layer.

The set of all weights defines a configuration of the CNN. In the learning phase, an initial configuration is adjusted based on the training data using a learning algorithm such as forward-backward("FB")-propagation or other optimization schemes.

The training mode is preferably supervised, that is, is based on annotated training data. Annotated training data includes pairs or training data items. For each pair, one item is the training input data and the other item is target training data known a priori to be correctly associated with its training input data item. This association defines the annotation and is preferably provided by a human expert. In training mode preferably multiple such pairs are applied to the input layer to propagate through the CNN until an output emerges at OL. Initially, the output is in general different from the target. During the optimization, the initial configuration is readjusted so as to achieve a good match between input training data and their respective target for all pairs.

More specifically, the input training data items are applied to the input layer (IL) and passed through a cascaded group(s) of convolutional layers CL1, CL2 and possibly one or more pooling layers PL1, PL2, and are finally passed to one or more fully connected layers. The convolutional module is responsible for feature based learning (e.g. identifying pain, respiratory drifts, amplitudes, body movement, etc.), while the fully connected layers are responsible for more abstract learning, for instance, the impact of the features on the image quality, and possible consequences for the imaging procedure such as the imaging pulse sequence in MRI. The impact on the imaging procedure is indicated by nodes in the output layer OL. In other words, the output layer OL includes the parameters for the fixation device FD and/or imager IA.

The exact grouping and order of the layers as per Fig. 7 is but one exemplary embodiment, and other groupings and order of layers are also envisaged in different embodiments. All that has been said above is of equal application to other NNs envisaged herein, such as fully connected classical perceptron type NNs, deep or not, and recurrent NNs, or others. In variance to the above, unsupervised learning or reinforced leaning schemes may also be envisaged in different embodiments.

The annotated training data, for the MLC as envisaged herein for use with CU in the SSI, may need to be reformatted into structured form. The annotated training data may be arranged as vectors or matrices or tensor (arrays of dimension higher than 2). This reformatting may be done by a data pre-processor module (not shown).

The MLC for present purposes may be configured as two separate NNs, preferably CNNs, connected in series.

One of the CNNs performs the controlling for the PM loop and the other for the IQ loop. The two CNNs may be referred to herein as the PM-CNN and IQ-CNN, respectively. In learning or deployment, output of the PM-CNN may be fed into the input of the IQ-loop. Alternatively, both CNNs may be consolidated into a single CNN architecture.

The training data for the PM-CNN comprises pairs of pain sensor readings associated with acceptable fixation device control parameters that are known to result in an acceptable pain level as per the pain threshold.

The training data for the IQ-CNN comprises pairs of fixation device control parameters associated with known imager control parameters that are known to yield imagery at acceptable IQ levels as per the IQ threshold.

The training data sets are applied to the two CNNs and processed according to a learning algorithm such as the FB-propagation algorithm as mentioned before. At the end of the training phase, the two pre-trained CNNs may then be used in deployment phase to operate the SSI as described to control both, the imager IA and the fixation device, as earlier described. In deployment, a pain level measurement from sensor(s) PS1,PS2 is obtained during normal use for therapy or imaging of a patient. The pain level measurement is applied to the input layer IL of the trained neural network. The pain measurement is then propagated through the PM-CNN to obtain the fixation device control parameters to control fixation device FD. The fixation device control parameter is then further propagated through the IQ-CNN to obtain parameters for the imaging procedure which are then applied to the imager, if different from the currently used imaging procedure.

The MLC helps to achieve optimal IQ also for not experienced users. During deployment, the machine learning algorithm in the MLC operates to decide on the often complex parameter settings, including fixation device control parameters and imaging procedure parameters.

During the learning phase, the mentioned fixation device versus pain level pair may be generated as follows. At random or according to a deterministic protocol, the fixation device is controlled by the CU to deliver a number of different pressure profiles, similarly as in the above mentioned pain locator mode. The pressure profile delivery may include forming repeated sweeps of pressure. The different profiles may include any or more of: constant pressure, pressure pulses or a triangular sweep, or any other pain profile shape. In this manner a relationship between a pressure history of the pressure signals and the pain signal and/or the resulting image quality can be established. The data so generated may be annotated by a user or automatically by pain threshold and or IQ threshold comparison.

The training data generation can be performed using an elastic phantom model of the anatomy of interest such as the breast. In addition or instead, experimental data using human imaging may be employed. Once the training data is so generated, the MLC is fed by this data and any of the above mentioned training algorithms may be used to adjust the parameters of the MLC. The MLC component is preferably arranged as a multi-core processor such as a GPU (graphical processing unit) or TPU (tensor processing unit).

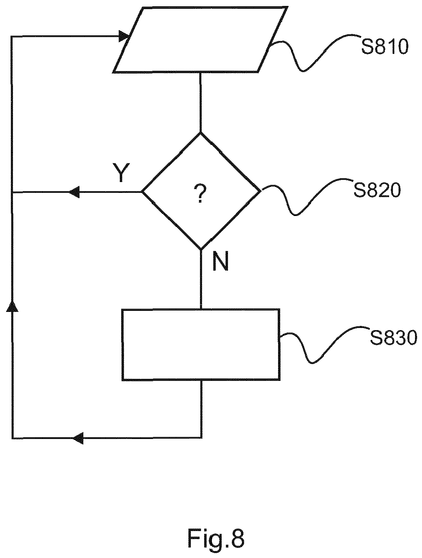

Reference is now made to Fig. 8 which shows a flow chart of a method of imaging support as envisaged herein. The method relates to operation of the above described system SSI but the following method steps may also be understood as a teaching in their own right, not necessarily tied to the architecture of embodiments in Figs. 1-7. Turning now first to Fig. 8, at step S810 a pain measurement signal is received from one or more pain sensors.

The pain measurements are taken in relation to an anatomic part of a patient such as a female human breast. The pain sensors may be arranged as in any of the above described embodiments in Figs. 1-7.

Preferably, but not necessarily, the pain sensor is included in a fixation device that is to hold the breast stationary and compress same during the imaging procedure.

The pain measurements are taken prior to an/or during an ongoing imaging procedure that is, whilst the imaging device exposes the breast to interrogating signals such as radio frequency pulse signal sequences in MRI imaging.

Again, although main reference will be made to MRI imaging of the human breast, other imaging modalities and/or other anatomic parts such as a human head, arm, leg, knee are also envisaged herein specifically.

At step S820 control signals are computed based on the measurement, particularly on the pain measurements. The control signals are to control the compression level exerted by the fixation device and/or the imaging procedure performed or to be performed by the imager IA.

The control signals computed at step S820 are computed to intervene or interact with the imaging apparatus during the imaging procedure and/or with the fixation device. In particular, both fixation device and the imaging apparatus are controlled by the one or more control signals.

The computing of the control signals at step S820 furthermore includes a decision whether or not to interact at all. If no interaction is necessary given for the current pain level as per the received measurements, then no interaction is taken.

If, however, it is decided that the measured pain level, as per the measurement necessitates interaction, the process flow moves to action intervention step S830 where action is taken to interact with either the imager or the fixation device or with both.

The interaction at step S830 may occur during the ongoing image procedure, that is, whilst image data is collected during exposure of the anatomic part to an interrogating signal as emitted by the imaging apparatus IS.

The interaction at step S830 is based on the control signals computed at step S820. The interaction may be organized as two linked feedback loops: the PM loop and the IQ loop. In the PM loop, pain level measurements are received at a suitable sampling rate and compared to pain threshold whilst the fixation device is readjusted to find a compression level and distribution so that the pain measurements do not, or no longer, exceed the pain threshold. The different fixation device readjustments may have consequences for the IQ and hence the imaging procedure. Therefore, the control signals to effect the fixation device readjustments are fed into the second, the IQ loop, to check whether the IQ threshold is still respected. If IQ threshold is not respected, the imaging procedure may be changed and/or the fixation device is readjusted again, thus, flow control links back again into the PL loop, and so forth.

The control signals at step S820 may be computed based on look-up tables, deterministic functional expressions or by using a pre-trained machine learning component as explained earlier with reference to Fig. 7.

The measured signals are input into the machine learning component MLC and a decision as to whether or not to intervene and the manner in which this intervention is to be realized is then output at the output layer of the machine learning component.

If the passage through the two loops results in deteriorating IQ, the interaction S830 may include suspending the currently running imaging procedure. Suspension may be required if the fixation device readjustment takes more than a preset time period or if the readjustments are more large scale. How the imaging procedure is to be changed may depend on the degree of deformation or compression. In MRI, there may be further such dependence on the status (time) of the current pulse sequence and the type of pulse sequence currently used (eg, T1-weighted, T2-weighted, etc).

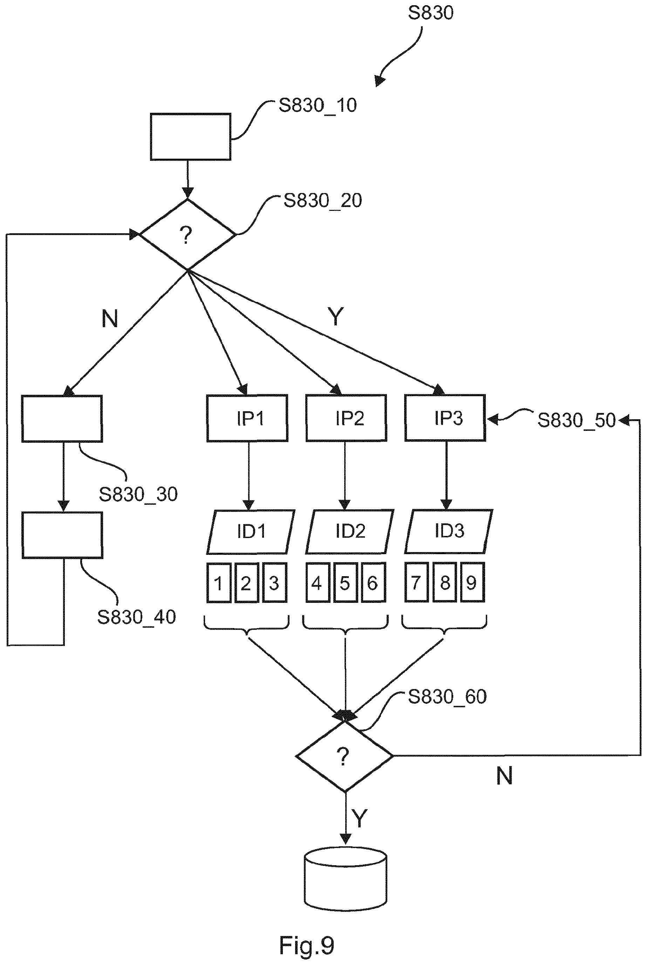

Reference is now made to Fig. 9 which shows in more detail embodiments of the interaction at step S830, in particular in case when imaging procedure is to be changed.

At step S830-10 an "OK" signal is received to start the imaging procedure once an initial fixation of the breast is realized by fixation device.

At step S830-20 pain signal measurements are continued to be received.

If it is decided that no intervention is necessary the imaging procedure continues at step S830-50 using a current set of imaging control parameters IP1 to acquire a first set of image data ID1 in a first run, comprising image data items (or frames), numbered schematically as "1-3" in Fig. 8.

If at one point in time it is decided at step S830-20 that the pain signal indicates a level where an interaction is warranted, a control signal is sent at step S830-30 to the imaging apparatus to request a change of the current imaging procedure. This request for a change may include in particular suspending the ongoing imaging procedure. Interaction with the imager may also include changing the imaging protocol or imaging strategy. For MRI, this may include changing the slice being imaged or changing the type or timing of a pulse sequence.

If it is decided in step S830-30 to interrupt the imaging, the compression level and/or the distribution of the pressure exerted by the fixation device on the breast is adjusted at step S830-40. If the readjustments are minor, imaging procedure may continue however.

During this time in the PM feedback loop, pain sensor measurements from the pressure sensor are still received at step S830-20. Once the pressure level measured drops under the pain threshold, thus indicating that the patient no longer experiences intolerable pain, process flow returns to step S830-50 so as to resume the image procedure IP2, possibly using different image parameters, to acquire a second set of image data ID2 comprising exemplary frames "4-6".

The process then continues in this manner until the imaging procedure is concluded and a complete set of image data ID1-ID3 has been acquired.

An optional global image quality step S830-60 is then used to decide whether the image data is consistent locally or globally to maintain the desired image quality.

If it is decided that the image quality can be maintained, the complete set ID1-ID3 of acquired image data is forwarded to the re-constructor 58 to reconstruct imagery which can then be stored, displayed or otherwise processed.

If, however, it is decided that there is no consistency, the whole or as subset, say ID2, may need to be re-acquired. A lack of consistency may be caused by using very different compression levels during the different imaging procedures ID1, ID2, ID separated by interruptions. In CT, the consistency check may be done through a scout scan to check whether the overall motion was severe enough to warrant a repeat of the whole or parts of the imaging procedure.

Preferably however it may not be necessary to acquire all image data but it may be decided to only reacquire a certain section, such as ID, but to maintain ID1 and ID3. Thus, the proposed method allows maintaining a certain pre-defined image quality whilst still reducing the discomfort and pain level experienced by the patient. Unnecessary re-acquisition of image data and, thus even further prolonging the imaging procedure, may be avoided. It may be understood that in particular in embodiments where the machine learning step is used at Step S820, the local consistency check step mentioned is implicit as the machine learning component has been trained to ensure IQ is respected. At least a global consistency check may still be performed.

In any one of the above described embodiments, instead of or in addition to the imaging apparatus IA, a therapy device, such as a linac or other, may be used and all of the above described principles equally apply to the therapy device. In these embodiments, the control signals then relate to controlling the linac for instance during radiation therapy delivery. Controlling the linac includes in particular control of a multi-leaf collimator such as in IMRT and/or movement of a treatment head of the linac around the region of interest to deliver a prescribed, spatially distributed amount of radiation dose. In addition or instead of IQ parameters, it is the treatment quality parameters (for instance, treatment plan parameters) that are monitored such as specific dose delivery to specific areas and the sparring of surrounding tissue, etc.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A system for supporting operation of an imaging or therapy apparatus, comprising:

   - an interface (IN) for receiving a pain measurement signal as measured by one or more sensors (S) in relation to an anatomic part (BR) of the patient (PAT) i) being imaged in an imaging procedure by the imaging apparatus or ii) being under therapy in a therapy procedure delivered by the therapy device, whilst the part (BR) is held in an adjustable fixation device (FD);
   - a control unit (CU) configured to process the pain measurement signal to compute at least one control signal; and
   - a control interface (CIF) configured to interact during the imaging or therapy procedure with i) the imaging apparatus (IA) or therapy device and/or ii) the fixation device (FD) based on the control signal to a) influence the imaging procedure or therapy procedure and/or b) to adjust the fixation device so as to change the manner in which the part (BR) is being held.

2. System of claim 1, wherein the control unit (CU) is configured to take into account at least one pre-set image quality or therapy quality parameter when computing the control signal.

3. System of claim 2, wherein the control unit (CU) is configured to compute the control signal so as to at least maintain the imaging quality as per the pre-set image or the therapy quality parameter.

4. System of any one of the previous claims, wherein the control unit (CU) is configured in at least one feedback architecture in relation to a pain threshold for the received pain measurement signal and/or the preset image or therapy quality parameter.

5. System of any one of the previous claims, wherein the imaging procedure includes acquisition of image data.

6. System of claim 5, wherein the influencing of the imaging procedure includes i) suspending or resuming the acquisition of image data or ii) suspending or resuming therapy delivery.

7. System of any one of the previous claims, wherein the fixation device (FD) is adjustable so as to change a pressure distribution exerted by the fixation device on at least a portion of the part.

8. System of any one of the previous claims, wherein the fixation device includes an adjustable contact surface urgeable into contact with at least a portion of the part, wherein the said contact surface shape is adjustable so as to conform to at least a shape of a portion of the part (B).

9. System of claim 8, wherein the contact surface shape is adjustable in 3D.

10. System of any one of the previous claims, wherein the control unit (CU) is configurable to operate in a pain locator mode so as to determine, based on the pain measurement signal, where in or on the part (BR) a pain is caused.

11. System of any one of the previous claims, wherein the imaging apparatus is any one of: an MRI imager, an X-ray imager; an emission imager, a linac system.

12. System of any one of the previous claims, comprising a pre-trained machine learning component (MLC) included in or, coupled to, the control unit (CU).

13. An imaging arrangement (AR), comprising: the system of any one of the previous claims, the imaging or therapy apparatus, the at least one pain sensor.

14. Method of supporting imaging or therapy, comprising:

- receiving (S810) a pain measurement signal as measured by one or more sensors (S) in relation to a part (BR) of the patient (PAT) i) being imaged in an imaging procedure by the imaging apparatus or ii) being under therapy in a therapy procedure delivered by the therapy device, whilst the part is held in an adjustable fixation device;
- processing (S820) the pain measurement signal to compute at least one control signal; and
- interacting (S830, S830-30) during the procedure with the i) imaging apparatus (IA) or therapy device and/or ii) the fixation device (FD) based on the control signal to a) influence the imaging or therapy procedure and/or b) to adjust the fixation device so as change the manner in which the part (BR) is being held.

15. A method of training an initial machine learning component to obtain the pre-trained machine learning component (MLC) of claim 12.

16. A computer program element, which, when being executed by at least one processing unit (PU), is adapted to cause the processing unit (PU) to perform the method as per claim 14 or 15.

17. A computer readable medium having stored thereon the program element of claim 16.

18. A fixation device (FD) configured to hold an anatomic part of a patient during imaging with an imaging apparatus or during delivery of a therapy by a therapy delivery apparatus, wherein the fixation device (FD) is adjustable so as to change a pressure distribution excreted by the fixation device on at least a portion of the part.

19. Fixation device of claim 18, wherein the fixation device includes an adjustable contact surface urgeable into contact with the part, wherein the said contact surface shape is adjustable so as to conform to at least a shape of a portion of the part (B).

20. Fixation device of any claim 18 or 19, wherein the contact surface is formed from a plurality of contact elements individually addressable to effect a deformation or motion of same, the elements together forming the contact surface.

Fig.1

Fig.2

Fig.3

Fig.4

EP 3 618 079 A1

Fig.5

Fig.5A

PCD                    UI                    CU

```
┌──────────────┐   ┌──────────────┐   ┌──────────────┐
│              │   │              │   │              │
│              │   │              │   │              │
└──────────────┘   └──────────────┘   └──────────────┘
       ↕                  ↕                  ↕
┌──────────────────────────────────────────────────────┐            IFFD
│                                                        │  ←──────
│                          FD                            │
│                                                        │
└──────────────────────────────────────────────────────┘
       ↕                  ↕                  ↕
┌──────────────┐   ┌──────────────┐   ┌──────────────┐
│              │   │              │   │              │
│              │   │              │   │              │
└──────────────┘   └──────────────┘   └──────────────┘

  IA                 PS1                   PS2
```

Fig.6

Fig.7

Fig.8

Fig.9

Europäisches
Patentamt

European
Patent Office

Office européen
des brevets

# EUROPEAN SEARCH REPORT

Application Number

EP 18 19 1668

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/272682 A1 (SHENG KE [US]) 1 October 2015 (2015-10-01) * paragraphs [0038] - [0041]; claim 1; figures 5A-7C * ----- | 18-20 | INV. G16H40/63 G16H30/20 G16H20/10 G16H20/40 |
| X | US 9 622 829 B2 (VOGELE MICHAEL [DE]; ISYS MEDIZINTECHNIK GMBH [AT]) 18 April 2017 (2017-04-18) * claim 1; figures 1-6 * * column 5, line 20 - line 24 * ----- | 18,19 | |
| X | WO 2014/109636 A1 (AMC AMSTERDAM [NL]) 17 July 2014 (2014-07-17) * claim 1; figure 1 * * page 2, line 27 - line 33 * * page 3, line 6 - line 14 * ----- | 1-17 | |
| A | US 2018/192940 A1 (SHIMAZU HIDEAKI [JP] ET AL) 12 July 2018 (2018-07-12) * claim 1 * ----- | 1-20 | |
| | | | TECHNICAL FIELDS SEARCHED (IPC) G16H |

The present search report has been drawn up for all claims

| Place of search | Date of completion of the search | Examiner |
|---|---|---|
| Munich | 5 February 2019 | Beligny, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 19 1668

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2019

| Patent document cited in search report | | | Publication date | Patent family member(s) | | | Publication date |
|---|---|---|---|---|---|---|---|
| US 2015272682 | A1 | | 01-10-2015 | US | 2015272682 | A1 | 01-10-2015 |
| | | | | WO | 2014074602 | A1 | 15-05-2014 |
| US 9622829 | B2 | | 18-04-2017 | DE | 202011005573 | U1 | 23-04-2012 |
| | | | | EP | 2699185 | A1 | 26-02-2014 |
| | | | | ES | 2694404 | T3 | 20-12-2018 |
| | | | | US | 2012266898 | A1 | 25-10-2012 |
| | | | | WO | 2012143142 | A1 | 26-10-2012 |
| WO 2014109636 | A1 | | 17-07-2014 | EP | 2943125 | A1 | 18-11-2015 |
| | | | | NL | 2010095 | C | 09-07-2014 |
| | | | | US | 2015297150 | A1 | 22-10-2015 |
| | | | | WO | 2014109636 | A1 | 17-07-2014 |
| US 2018192940 | A1 | | 12-07-2018 | CN | 108024800 | A | 11-05-2018 |
| | | | | EP | 3175796 | A1 | 07-06-2017 |
| | | | | JP | 6122927 | B2 | 26-04-2017 |
| | | | | JP | 2017064277 | A | 06-04-2017 |
| | | | | KR | 20170049463 | A | 10-05-2017 |
| | | | | US | 2018192940 | A1 | 12-07-2018 |
| | | | | WO | 2017056532 | A1 | 06-04-2017 |

EPO FORM P0459

For more details about this annex : see Official Journal of the European Patent Office, No. 12/82